# EUROPEAN PATENT SPECIFICATION

(11) **EP 4 071 135 B1**
(45) Date of publication and mention of the grant of the patent: **27.11.2024**
(21) Application number: 19955221.7
(22) Date of filing: 27.12.2019
(51) Int. Cl.: C07C 319/22, C07C 323/59, C07K 7/06, C07K 1/06, C07K 1/04

(54) **ETELCALCETIDE INTERMEDIATE AND METHOD FOR SYNTHESIZING ETELCALCETIDE**
ETELCALCETID-ZWISCHENPRODUKT UND VERFAHREN ZUM SYNTHETISIEREN VON ETELCALCETID
INTERMÉDIAIRE D'ÉTELCALCÉTIDE ET PROCÉDÉ DE SYNTHÈSE D'ÉTELCALCÉTIDE

(30) Priority: 03.12.2019 CN 201911217130
(43) Date of publication of application: 12.10.2022
(73) Proprietor: Asymchem Laboratories (Tianjin) Co., Ltd., Tianjin 300457 (CN)
(72) Inventor: LI, Jiuyuan, Tianjin 300457 (CN); MATT, Johnson, Morrisville, North Carolina 27560 (US); LI, Changfeng, Tianjin 300457 (CN); JING, Lutao, Tianjin 300457 (CN); BALASUBRAMANIAN, Arumugam, Tianjin 300457 (CN); LEI, Xiaolong, Tianjin 300457 (CN); ZHU, Zili, Tianjin 300457 (CN)
(74) Representative: Glawe, Delfs, Moll
(86) International application number: PCT/CN2019/129440
(87) International publication number: WO 2021/109297

(56) References cited:
- WO-A1-2017/114238
- WO-A1-2017/114238
- ZUBERI S ET AL: "Synthesis of Asymmetric Cystines", TETRAHEDRON LETTERS, ELSEVIER, AMSTERDAM , NL, vol. 39, no. 41, 8 October 1998 (1998-10-08), pages 7567 - 7570, XP004134353, ISSN: 0040-4039, DOI: 10.1016/S0040-4039(98)01614-1

## Description

### Technical Field

The present disclosure relates to the technical field of chemical synthesis, in particular to an etelcalcetide intermediate and a method for synthesizing etelcalcetide.

### Background

Etelcalcetide is a novel calcimimetic agent developed by Kai Pharmaceuticals, Inc. that may inhibit the secretion of a parathyroid hormone. The etelcalcetide may bind and activate a calciumsensing receptor on a parathyroid gland, and the reduction in parathyroid hormone level is achieved.

The etelcalcetide is composed of three *D-* arginines, two *D-* alanines, one *D*-arginine amide, one *L*-cysteine and one *D*-cysteine (N-terminal blocked by an acetyl group), herein the *D*-cysteine and the *L*-cysteine are linked together by a disulfide bond (*N*-acetyl-*D*-cysteinyl-*D*-alanyl-*D*-arginyl-*D*-arginyl-*D*-arginyl-*D*-alanyl-*D*-Argininamide,disulfidewithL-cysteine).

In currently disclosed patents, they are all linked to 7 peptides in solid-phase, and then choose different methods to link the disulfide bond, especially in the process of removing Methylcyclopentadienyl-Manganese-Tricarbony (MMT), it is necessary to use 1%-2% of Trifluoroacetic Acid (TFA)/Dichloromethane (DCM) to repeat an operation by more than 15 times, and the operation is quite complicated.

Patent WO2017114238 A1: a liquid-phase synthesis method adopted has a long route and the total yield is only 11%; Patent WO 2017/114240 A1: according to the patent, the purity of a crude peptide is 81.0%, there are many impurities, and the total yield after purification is 30.5%; Patent US 2019/0100554 A1: according to the patent, the purity of the crude peptide is relatively low, it needs to be purified by many times, and the total yield is 50%; and in Chinese Patent CN201811277081, the purity of the crude peptide is 88.2%, and the total yield after purification is 57.8%. In other words, an existing method of solid-phase synthesis of 7-peptide-linked cysteine and derivatives thereof has problems such as the low purity of the crude peptide, many impurities, and difficulty in purification.

### Summary

The present disclosure aims to provide an etelcalcetide intermediate and a method for synthesizing etelcalcetide. The intermediate is used for synthesizing the etelcalcetide, as to solve a technical problem in an existing technology that the etelcalcetide synthesis process is complicated.

In order to achieve the above purpose, according to one aspect of the present disclosure, a method for synthesizing an etelcalcetide intermediate is provided. The etelcalcetide intermediate is Fmoc-*D*-Cys(S-S-(N-Boc)-*L*-Cys(OtBu))-OH, and a structural formula I thereof is as follows:

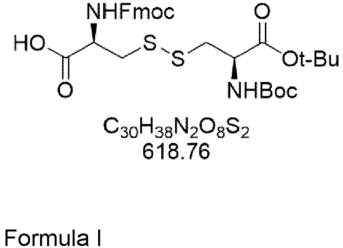

The method for synthesizing the etelcalcetide intermediate includes the following steps: *N*-Boc-*L*-Cys-OtBu is taken as a starting raw material, and a primary product 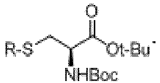 is generated through a substitution reaction, herein R is S-Py or Cl, and the primary product performs a coupling reaction with Fmoc-*D*-Cys-OH amino acid to obtain Fmoc-*D*-Cys(S-S-(*N*-Boc)-*L*-Cys(OtBu))-OH.

Further, the primary product is Py-S-S-(*N*-Boc)-*L*-Cys-OtBu, and Fmoc-*D*-Cys(S-S-(*N*-Boc)-*L-*Cys(OtBu))-OH is prepared by the following steps: *N*-Boc-*L*-Cys-OtBu performs a substitution reaction with dithiodipyridine to synthesize and obtain a primary product Py-S-S-(*N*-Boc)-*L*-Cys-OtBu; and Py-S-S-(*N*-Boc)-*L*-Cys-OtBu is coupled with Fmoc-*D*-Cys-OH to obtain Fmoc-*D*-Cys-(S-S-(*N-*Boc)-*L*-Cys-OtBu)-OH.

Further, Fmoc-*D*-Cys(S-S-(*N*-Boc)-*L*-Cys(OtBu))-OH is prepared by the following steps: at a room temperature, *N*-Boc-*L*-Cys-OtBu and dithiodipyridine are added to a solvent A, and stirred for 6-12 h, then water is added, an extractant is used to extract, an obtained organic phase is dried and filtered, and after purification, Py-S-S-(*N*-Boc)-*L*-Cys-OtBu is obtained; Fmoc-*D*-Cys-OH and Py-S-S-(*N*-Boc)-*L*-Cys-OtBu are added to a solvent B, the temperature is controlled at 15-30 °C and it is stirred and reacted for 0.5-2 h, the reaction system is water-washed, concentrated and purified to obtain Fmoc-*D*-Cys(S-S-(*N*-Boc)-*L*-Cys(OtBu))-OH; preferably, the solvent A is selected from one or more of N,N-dimethylformamide (DMF), N-methylpyrrolidone (NMP) and N,N-dimethylacetamide (DMAc); preferably, the extractant is selected from one or more of ethyl acetate (EtOAc), methyl tert-butyl ether (MTBE) and dichloromethane (DCM); and preferably, the solvent B is selected from one or more of DCM, DMF, tetrahydrofuran (THF), NMP and DMAc.

Further, in the reaction between N-Boc-*L*-Cys-OtBu and dithiodipyridine, the molar ratio of N-Boc-*L*-Cys-OtBu and dithiodipyridine is 1:1.2-1:6.4; the concentration of *N*-Boc-*L*-Cys-OtBu in the solvent A is 0.01-0.3 g/mL; the concentration of Fmoc-*D*-Cys-OH in the solvent B is 0.01-0.3 g/mL; and the molar ratio of Fmoc-*D*-Cys-OH and Py-S-S-(*N*-Boc)-*L*-Cys-OtBu is 1:0.8-1.4.

Further, the primary product is (*N*-Boc)-L-Cys(S-Cl)-OtBu, and Fmoc-*D*-Cys(S-S-(*N*-Boc)-*L-*Cys(OtBu))-OH is prepared by the following steps: N-Boc-*L*-Cys-OtBu reacts with NCS to synthesize (*N*-Boc)-*L*-Cys(S-Cl)-OtBu; and (*N*-Boc)-*L*-Cys(S-Cl)-OtBu reacts with Fmoc-*D*-Cys-OH to obtain Fmoc-*D*-Cys(S-S-(*N*-Boc)-*L*-Cys(OtBu))-OH.

Further, Fmoc-*D*-Cys(S-S-(*N*-Boc)-*L*-Cys(OtBu))-OH is prepared by the following steps: A. *N-*Boc-*L*-Cys-OtBu is dissolved in a solvent C, the temperature is controlled at 0-10°C, N,N-diisopropylethylamine (DIPEA) is added, and N-chlorosuccinimide (NCS) is added in batches, it is stirred for 4-5 h, after the reaction, it is filtered, and rinsed to obtain a filtrate; and B. the temperature is controlled at 0-10°C, Fmoc-*D*-Cys-OH is added to the filtrate, and DIPEA is added, the reaction temperature is controlled at 10-30°C, and it is stirred for 0.5-2 h; the reaction system is water-washed, concentrated and purified to obtain Fmoc-*D*-Cys(S-S-(*N*-Boc)-*L*-Cys(OtBu))-OH; and preferably, the solvent C is selected from one or more of DCM, THF, DMF NMP and DMAc.

Further, in the step A, *N*-Boc-*L*-Cys-OtBu is dissolved in the solvent C to obtain solution with a concentration of 0.01-0.3 g/mL, the amount of DIPEA added is 2-3 eq of moles, and the amount of NCS added is 1.1-1.5 eq; and in the step B, the amount of Fmoc-*D*-Cys-OH added is 1.1-1.5 eq.

According to another aspect of the present disclosure, a method for synthesizing etelcalcetide is provided. The synthesis method includes the following steps: S1, Fmoc-*D*-Cys(S-S-(*N*-Boc)-*L-*Cys(OtBu))-OH is synthesized by any one of the methods for synthesizing the etelcalcetide intermediates; and S2, NH₂-*D*-Ala-*D*-Arg-*D*-Arg-*D*-Arg-*D*-Ala-*D*-Arg reacts with Fmoc-*D*-Cys(S-S-(*N-*Boc)-*L*-Cys(OtBu))-OH, and Fmoc is removed, and then acetylation is performed to obtain the etelcalcetide.

Further, in the S2, NH₂-*D*-Ala-*D*-Arg-*D*-Arg-*D*-Arg-*D*-Ala-*D*-Arg is NH₂-*D*-Ala-*D*-Arg-*D*-Arg-*D-*Arg-*D*-Ala-*D*-Arg-resin hexapeptide.

Further, the S2 includes: an amino resin is used to link NH₂*-D*-Ala-*D*-Arg-*D*-Arg-*D*-Arg-*D*-Ala-*D-*Arg-resin hexapeptide according to a method of solid-phase synthesis, Fmoc-*D*-Cys(S-S-(*N*-Boc)-*L-*Cys(OtBu))-OH, benzotriazol-1-yl-oxytripyrrolidino-phosphonium hexafluorophosphate (PyBop) and DIPEA are activated at 0-5°C for 0-10 min, the temperature is controlled at 20~30°C and it is reacted for 2-6 h. After the reaction, it is washed by DMF for 4-6 times, Fmoc is removed by 10%-20% of piperidine, and then the acetylation is performed to obtain a peptide resin of the etelcalcetide.

Further, the ratio of Fmoc-*D*-Cys(S-S-(*N*-Boc)-*L*-Cys(OtBu))-OH:PyBop:DIPEA is 3:3-6:3-6.

A technical scheme of the present disclosure is applied, by synthesizing the key intermediate Fmoc-*D*-Cys(S-S-(*N*-Boc)-*L*-Cys(OtBu))-OH, and then synthesizing the etelcalcetide, a side reaction while a peptide chain constructs a disulfide bond is avoided, the operation is simplified, and the purity and yield may be improved while the etelcalcetide is synthesized. It is important that raw materials for synthesizing this intermediate are cheap and easy to obtain, and the process is simple.

### Brief Description of the Drawings

Drawings constituting a part of the present disclosure are used to provide further understanding of the present disclosure, and exemplary embodiments of the present disclosure and descriptions thereof are used to explain the present disclosure, and do not constitute improper limitation to the present disclosure.
Fig. 1 shows a purity map of Fmoc-*D*-Cys(S-S-(*N*-Boc)-*L*-Cys(OtBu))-OH in Embodiment 1;
Fig. 2 shows an liquid chromatography mass spectrometry (LCMS) spectrum of an intermediate Fmoc-*D*-Cys(S-S-(*N*-Boc)-*L*-Cys(OtBu))-OH of Embodiment 1 (LC-MS: m/z=617.9 (M-1,30ev), 1235.7(2M-1,30ev)); and
Fig. 3 shows a purity map of purified etelcalcetide prepared in Embodiment 3.

### Detailed Description of the Embodiments

It should be noted that embodiments in the present disclosure and features of the embodiments may be combined with each other in the case without conflicting. The present disclosure is described in detail below with reference to the drawings and in combination with the embodiments.

Abbreviations involved in the present disclosure are explained as follows:
Fmoc: 9-fluorenemethoxycarbonyl.
Boc: tert-butylcarbonyl.
tBu: tert-butyl.
Arg: arginine.
Cys: cysteine.
Ala: alanine.
PyBop: benzotriazol-1-yl-oxytripyrrolidino-phosphonium hexafluorophosphate.
DIPEA: N,N-diisopropylethylamine.
DCM: dichloromethane.
NCS: N-chlorosuccinimide.
DMF: N,N-dimethylformamide.
NMP: N-methylpyrrolidone.
DMAc: N,N-dimethylacetamide.
THF: tetrahydrofuran.
OtBu: tert-butoxy.
EtOAc: ethyl acetate.

In the synthesis of etelcalcetide, the most critical is the synthesis of a disulfide bond. The yield of this step directly affects the final yield of the entire route. It is found by the inventor of the present application that in the synthesis of a key intermediate Fmoc-*D*-Cys(S-S-(*N*-Boc)-*L*-Cys(OtBu))-OH (N-fluorenylcarbonyl-*D*-cysteine-S-S-(N-tert-butoxycarbonyl-L-cysteine tert-butyl ester)), the synthesis of the disulfide bond in advance may perfectly avoid problems of low efficiency and poor yield of a solid-phase reaction.

According to a typical embodiment of the present disclosure, a method for synthesizing an etelcalcetide intermediate is provided. The etelcalcetide intermediate is Fmoc-*D*-Cys(S-S-(*N*-Boc)-*L*-Cys(OtBu))-OH, and the method for synthesizing the etelcalcetide intermediate includes the following steps: N-Boc-L-Cys-OtBu is taken as a starting raw material, and a primary product 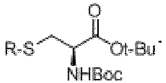 is generated through a substitution reaction, herein R is S-Py or Cl, and the primary product performs a coupling reaction with Fmoc-*D*-Cys-OH amino acid to obtain Fmoc-*D*-Cys(S-S-(*N*-Boc)-*L*-Cys(OtBu))-OH.

A technical scheme of the present disclosure is applied, by synthesizing the key intermediate Fmoc-*D*-Cys(S-S-(*N*-Boc)-*L*-Cys(OtBu))-OH, and then synthesizing the etelcalcetide, a side reaction while a peptide chain constructs a disulfide bond is avoided, the operation is simplified, and the purity and yield may be improved while the etelcalcetide is synthesized. It is important that raw materials for synthesizing this intermediate are cheap and easy to obtain, and the process is simple.

According to a typical embodiment of the present disclosure, the primary product is Py-S-S-(*N-*Boc)-*L*-Cys-OtBu, and Fmoc-*D*-Cys(S-S-(*N*-Boc)-*L*-Cys(OtBu))-OH is prepared by the following steps: N-Boc-L-Cys-OtBu performs a substitution reaction with dithiodipyridine to synthesize and obtain a primary product Py-S-S-(*N*-Boc)-*L*-Cys-OtBu; and Py-S-S-(*N*-Boc)-*L*-Cys-OtBu is coupled with Fmoc-*D*-Cys-OH to obtain Fmoc-*D*-Cys-(S-S-(*N*-Boc)-*L*-Cys-OtBu)-OH. It is important that the raw materials for synthesizing this intermediate are cheap and easy to obtain, and the process is simple.

Preferably, Fmoc-*D*-Cys(S-S-(*N*-Boc)-*L*-Cys(OtBu))-OH is prepared by the following steps: at a room temperature, *N*-Boc-*L*-Cys-OtBu and dithiodipyridine are added to a solvent A, and stirred for 6-12 h, then water is added, an extractant is used to extract, an obtained organic phase is dried and filtered, and after purification, Py-S-S-(*N*-Boc)-*L*-Cys-OtBu is obtained; Fmoc-*D*-Cys-OH and Py-S-S-(*N*-Boc)-*L*-Cys-OtBu are added to a solvent B, the temperature is controlled at 15-30 °C and it is stirred and reacted for 0.5-2 h, a reaction system is water-washed, concentrated and purified to obtain Fmoc-*D*-Cys(S-S-(*N*-Boc)-*L*-Cys(OtBu))-OH; preferably, the solvent A is selected from one or more of DMF, NMP and DMAc; preferably, the extractant is selected from one or more of eEtOAc, MTBE and DCM; and preferably, the solvent B is selected from one or more of DCM, DMF, THF, NMP and DMAc.

In order to improve the utilization rate of the raw materials and guarantee the rapid and effective progress of the reaction, further preferably, in the reaction between *N*-Boc-*L*-Cys-OtBu and dithiodipyridine, the molar ratio of *N*-Boc-*L*-Cys-OtBu and dithiodipyridine is 1:1.2-1:6.4; the concentration of *N*-Boc-*L*-Cys-OtBu in the solvent A is 0.01-0.3 g/mL; the concentration of Fmoc-*D-*Cys-OH in the solvent B is 0.01-0.3 g/mL; and the molar ratio of Fmoc-*D*-Cys-OH and Py-S-S-(*N-*Boc)-*L*-Cys-OtBu is 1:0.8-1.4.

According to a typical embodiment of the present disclosure, the primary product is (*N*-Boc)-*L-*Cys(S-CI)-OtBu, and Fmoc-*D*-Cys(S-S-(*N*-Boc)-*L*-Cys(OtBu))-OH is prepared by the following steps: *N*-Boc-*L*-Cys-OtBu reacts with NCS to synthesize (*N*-Boc)-*L*-Cys(S-Cl)-OtBu; and (*N*-Boc)-*L*-Cys(S-CI)-OtBu reacts with Fmoc-*D*-Cys-OH to obtain Fmoc-*D*-Cys(S-S-(*N*-Boc)-*L*-Cys(OtBu))-OH.

Preferably, Fmoc-*D*-Cys(S-S-(*N*-Boc)-*L*-Cys(OtBu))-OH is prepared by the following steps: A. *N*-Boc-*L*-Cys-OtBu is dissolved in a solvent C, the temperature is controlled at 0-10°C, DIPEA is added, and NCS is added in batches, it is stirred for 4-5 h, after the reaction, it is filtered, and rinsed to obtain a filtrate; and B. the temperature is controlled at 0-10°C, Fmoc-D-Cys-OH is added to the filtrate, and DIPEA is added, the reaction temperature is controlled at 10-30°C, and it is stirred for 0.5-2 h; the reaction system is water-washed, concentrated and purified to obtain Fmoc-*D*-Cys(S-S-(*N*-Boc)-*L*-Cys(OtBu))-OH; and preferably, the solvent C is selected from one or more of DCM, THF, DMF NMP and DMAc.

In order to improve the utilization rate of the raw materials and guarantee the rapid and effective progress of the reaction, further preferably, in the step A, *N*-Boc-*L*-Cys-OtBu is dissolved in the solvent C to obtain solution with a concentration of 0.01-0.3 g/mL, the amount of DIPEA added is 2-3 eq of moles, and the amount of NCS added is 1.1-1.5 eq; and in the step B, the amount of Fmoc-*D*-Cys-OH added is 1.1-1.5 eq.

According to a typical embodiment of the present disclosure, a method for synthesizing etelcalcetide is provided. The synthesis method includes the following steps: S1, Fmoc-*D*-Cys(S-S-(*N*-Boc)-L-Cys(OtBu))-OH is synthesized by any one of the methods for synthesizing the etelcalcetide intermediates; and S2, NH₂-*D*-Ala-*D*-Arg-*D*-Arg-*D*-Arg-*D*-Ala-*D*-Arg reacts with Fmoc-*D*-Cys(S-S-(*N*-Boc)-L-Cys(OtBu))-OH, and Fmoc is removed, and then acetylation is performed to obtain the etelcalcetide.

Because the raw materials of the key intermediate Fmoc-*D*-Cys(S-S-(*N*-Boc)-*L*-Cys(OtBu))-OH are cheap and easy to obtain, and the process is simple, it also directly makes the raw materials for the synthesis method of the etelcalcetide in the present disclosure cheap and easy to obtain, and the process is simple.

Preferably, in the S2, NH₂-*D*-Ala-*D*-Arg-*D*-Arg-*D*-Arg-*D*-Ala-*D*-Arg is NH2-*D*-Ala-*D*-Arg-*D*-Arg-*D*-Arg-*D*-Ala-*D*-Arg-resin hexapeptide. Preferably, the S2 includes: an amino resin is used to link NH2-*D*-Ala-*D*-Arg-*D*-Arg-*D*-Arg-*D*-Ala-*D*-Arg-resin hexapeptide according to a method of solid-phase synthesis, Fmoc-*D*-Cys(S-S-(*N*-Boc)-*L*-Cys(OtBu))-OH, PyBop and DIPEA are activated at 0-5°C for 0-10 min, the temperature is controlled at 20~30°C and it is reacted for 2-6 h. After the reaction, it is washed with DMF for 4~6 times, Fmoc is removed by 10%-20% of piperidine, and then the acetylation is performed to obtain a peptide resin of the etelcalcetide. More preferably, the ratio of Fmoc-D-Cys(S-S-(N-Boc)-L-Cys(OtBu))-OH:PyBop:DIPEA is 3:3-6:3-6.

The beneficial effects of the present disclosure are further described below in combination with the embodiments.

Specific synthetic routes of the embodiments:

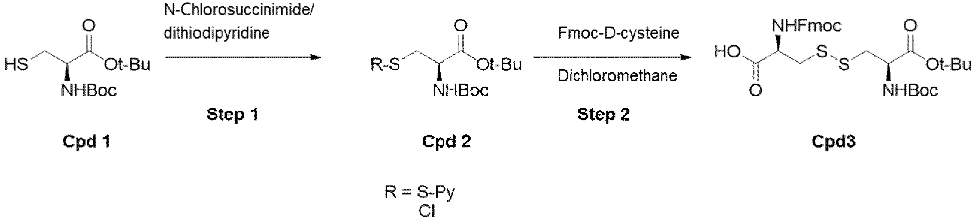

### Embodiment 1

### R=S-Py

*N*-Boc-*L*-Cys-OtBu(Cpd 1) is used as a raw material, and Py-S-S-(*N*-Boc)-*L*-Cys-OtBu(Cpd 2a) is synthesized by reacting *N*-Boc-*L*-Cys-OtBu(Cpd 1) with dithiodipyridine; and Py-S-S-(*N*-Boc)-*L-*Cys-OtBu(Cpd 2a) is coupled with Fmoc-*D*-Cys-OH to obtain Fmoc-*D*-Cys-(S-S-(*N*-Boc)-*L*-Cys-OtBu)-OH(Cpd 3).

### Step 1

At a room temperature, Cpd 1 (1.6 g, 1.0 eq) and 2,2-dithiodipyridne (5.1 g, 4.0 eq) are added to DMF (16 mL, 10 vol.). It is reacted and stirred at the room temperature for 6-12 h; then water is added to a system; ethyl acetate (100 mL*3) is used to extract; organic phases are dried by using MgSO₄ after being combined, and filtered; and the organic phase is concentrated to obtain a crude product Cpd 2a, and it is purified by a column chromatography to obtain pure Py-S-S-(*N*-Boc)-*L*-Cys-OtBu(Cpd 2a).

### Step 2

At 15~30°C, Fmoc-*D*-Cys-OH (1.0 g, 1.0 eq.) is added to DCM (30 ml, 30 vol.), then Cpd 2a (1.13 g, 1.0 eq.) is added to a reaction system; the temperature is controlled at 15-30°C, it is stirred and reacted for 0.5-2 h; the system is washed with water for 3 times, and concentrated to obtain a crude product Cpd 3; and pure Fmoc-*D*-Cys(S-S-(*N*-Boc)-*L*-Cys-(OtBu))-OH(Cpd3) is obtained by the column chromatography.

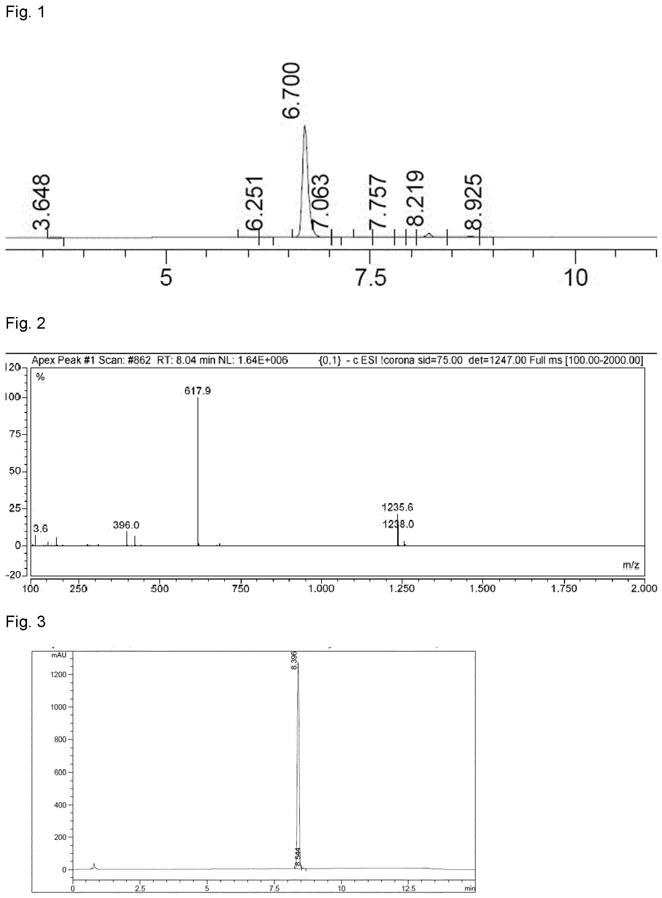

Fig. 1 shows a purity map of purified Cpd 3; and Fig. 2 shows an LCMS spectrum of purified Cpd 3.

### Embodiment 2

### R=CI

*N*-Boc-*L*-Cys-OtBu(Cpd 1) is used as a raw material, and (*N*-Boc)-*L*-Cys(S-Cl)-OtBu(Cpd 2b) is synthesized by reacting with NCS; and (*N*-Boc)-*L*-Cys(S-Cl)-OtBu(Cpd 2b) reacts with Fmoc-*D*-Cys-OH to obtain Fmoc-*D*-Cys(S-S-(*N*-Boc)-L-Cys-(OtBu))-OH(Cpd 3).

### Step 1

(*N*-Boc)-Cys-OtBu (0.28 g, 0.1 mmol) is dissolved in DCM (20 mL), it is stirred and the temperature is controlled at 0-10°C, and DIPEA (0.19 g, 0.15 mmol) is added; the temperature is controlled at 0-10°C and NCS (0.15 g, 1.1 eq) is added in batches; the reaction temperature is kept and it is stirred for 4-5 h, and an end point of the reaction is monitored by a high performance liquid chromatography (HPLC); after the reaction, it is filtered; a filter cake is rinsed with DCM (20 mL); and a filtrate is directly used for the next step after being combined.

### Step 2

The temperature is controlled at 0-10 °C, and Fmoc-*D*-Cys-OH (0.34 g, 0.1 mmol) is added to the filtrate of the previous step. DIPEA (0.15 g, 1.1 eq) is dropwise added to a reaction system; the reaction temperature is controlled at 10-30°C, and it is stirred for 0.5-2 h; the system is washed with water for 3 times, and concentrated to obtain a crude product Cpd 3, and pure Fmoc-*D*-Cys(S-S-(*N-*Boc)-*L*-Cys-(OtBu))-OH(Cpd3) is obtained by a column chromatography.

An LCMS spectrum of purified Cpd 3 is the same as that in Fig. 2.

### Embodiment 3

Synthesis of etelcalcetide using key intermediate:
An amino resin (including but not limited to a Sieber resin, Rink Amide MBHA, and a Rink Amide resin) is used to link NH₂-*D*-Ala-*D*-Arg-*D*-Arg-*D*-Arg-*D*-Ala-*D*-Arg-resin hexapeptide according to a method of solid-phase synthesis, a key intermediate is used to activate at 0-5°C for 0-5 min according to the ratio of Fmoc-*D*-Cys(S-S-(*N*-Boc)-L-Cys(OtBu))-OH:PyBop:DIPEA=3:3:3, the temperature is controlled at 20-30°C for 2-6 h, and a reaction end point is detected by kaiser. After the reaction, it is washed with DMF for 6 times, Fmoc is removed by 20% of piperidine, and then acetylation is performed to obtain a peptide resin of the etelcalcetide. The purity of a crude peptide after cleavage is 90.3%, the purity after preparation and purification is 99.51%, and the total yield is 65%.

Referring to the newly developed synthesis process of the etelcalcetide key intermediate Fmoc-*D*-Cys(S-S-(*N*-Boc)-L-Cys(OtBu))-OH, 100 g of a cysteine is used as a starting material, and the finally synthesized key intermediate is 357 g. This intermediate is used to synthesize the etelcalcetide according to Embodiment 3. After preparation and purification, 227 g of a pure product (TFA salt) is obtained, the purity is 99.51% (Fig. 3), and the total yield is 65%.

From the above description, it may be seen that the above embodiments of the present disclosure achieve the following technical effects: in the present application, a key intermediate Fmoc-D-Cys(S-S-(N-Boc)-L-Cys(OtBu))-OH of the etelcalcetide is firstly synthesized, this intermediate is used to synthesize the etelcalcetide, the purity may reach 99.51% after preparation (Fig. 3), and the separation yield is 73%, it solves a problem in an existing synthesis process that a reaction of disulfide bond mismatch exists between polypeptide chains, and there are many types of side reactions and by-products.

## Claims

1. A synthetic method for an etelcalcetide intermediate, wherein the etelcalcetide intermediate is Fmoc-*D*-Cys(S-S-Boc-*L*-Cys(OtBu))-OH, and the synthetic method for the etelcalcetide intermediate comprises the following steps: using *N*-(Boc)-*L*-Cys-OtBu as a raw material, and generating a primary product 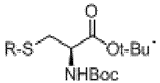 through a substitution reaction, wherein R is S-Py or Cl, enabling the primary product to perform a coupling reaction with a Fmoc-*D*-Cys-OH amino acid, as to obtain the Fmoc-*D*-Cys(S-S-Boc-*L*-Cys(OtBu))-OH;
when R is S-Py, the primary product is Py-S-S-Boc-*L*-Cys-OtBu, enabling the *N*-(Boc)-*L*-Cys-OtBu to perform the substitution reaction with dithiodipyridine to obtain a primary product Py-S-S-Boc-*L*-Cys-OtBu;
when R is Cl, the primary product is *N*-(Boc)-*L*-Cys(S-Cl)-OtBu, enabling *N*-(Boc)-*L*-Cys-OtBu to react with NCS to synthesize *N*-(Boc)-*L*-Cys(S-Cl)-OtBu.

2. The synthetic method of claim 1, wherein the primary product is Py-S-S-Boc-*L*-Cys-OtBu, and the Fmoc-*D*-Cys(S-S-Boc-*L*-Cys(OtBu))-OH is prepared by the following steps:
enabling the *N*-(Boc)-*L*-Cys-OtBu to perform the substitution reaction with dithiodipyridine to obtain a primary product Py-S-S-Boc-*L*-Cys-OtBu; and
enabling the Py-S-S-Boc-*L*-Cys-OtBu to be coupled with Fmoc-*D*-Cys-OH to obtain the Fmoc-*D*-Cys(S-S-Boc-*L*-Cys(OtBu)-OH.

3. The synthetic method of claim 2, wherein the Fmoc-*D*-Cys(S-S-Boc-*L*-Cys(OtBu))-OH is prepared by the following steps:
at a room temperature, adding the *N*-(Boc)-*L*-Cys-OtBu and the dithiodipyridine to a solvent A, stirring for 6-12 h, and adding water, extracting by using an extraction agent, drying and filtering an obtained organic phase, to obtain the Py-S-S-Boc-*L*-Cys-OtBu after purifying;
adding the Fmoc-*D*-Cys-OH and the Py-S-S-Boc-*L*-Cys-OtBu to a solvent B, controlling a temperature at 15-30 °C, stirring and reacting for 0.5-2 h, washing, concentrating and purifying a reaction system, as to obtain the Fmoc-*D*-Cys(S-S-Boc-*L*-Cys(OtBu))-OH.

4. The synthetic method of claim 3, wherein the solvent A is selected from one or more of DMF, NMP or DMAc;.

5. The synthetic method of claim 3, wherein the extraction agent is selected from one or more of EtOAc, MTBE or DCM.

6. The synthetic method of claim 3, wherein the solvent B is selected from one or more of DCM, DMF, THF, NMP or DMAc.

7. The synthetic method of claim 3, wherein in the reaction of the *N*-(Boc)-*L*-Cys-OtBu and the dithiodipyridine, a mole ratio of the *N*-(Boc)-*L*-Cys-OtBu and the dithiodipyridine is 1:1.2-1:6.4; a concentration of the *N*-(Boc)-*L*-Cys-OtBu in the solvent A is 0.01-0.3 g/mL; a concentration of the Fmoc-*D*-Cys-OH in the solvent B is 0.01-0.3 g/mL; and a mole ratio of the Fmoc-*D*-Cys-OH and the Py-S-S-Boc-*L*-Cys-OtBu is 1:0.8-1.4.

8. The synthetic method of claim 1, wherein the primary product is *N*-(Boc)-*L*-Cys(S-Cl)-OtBu, and the Fmoc-*D*-Cys(S-S-Boc-*L*-Cys(OtBu))-OH is prepared by the following steps:
enabling *N*-(Boc)-*L*-Cys-OtBu to react with NCS to synthesize *N*-(Boc)-*L*-Cys(S-Cl)-OtBu; and
enabling the *N*-(Boc)-L-Cys(S-Cl)-OtBu to react with the Fmoc-*D*-Cys-OH to obtain the Fmoc-*D*-Cys(S-S-Boc-*L*-Cys(OtBu))-OH.

9. The synthetic method of claim 8, wherein the Fmoc-*D*-Cys(S-S-Boc-*L*-Cys(OtBu))-OH is prepared by the following steps:
A. dissolving Boc-*L*-Cys-OtBu in a solvent C, controlling a temperature at 0-10 °C, adding DIPEA, and adding NCS in batches, stirring for 4-5 h, after ending a reaction, filtering, and eluting, to obtain filtrate;
B. controlling a temperature at 0-10 °C, adding the Boc-*L*-Cys-OtBu to the filtrate, adding DIPEA, reacting and controlling the temperature at 10-30 °C, stirring for 0.5-2 h; and washing, concentrating and purifying a reaction system to obtain the Fmoc-*D*-Cys(S-S-Boc-*L*-Cys(OtBu))-OH.

10. The synthetic method of claim 9, wherein the solvent C is selected from one or more of DCM, THF, DMF, NMP or DMAc.

11. The synthetic method of claim 9, wherein in the step A, dissolving the Boc-*L*-Cys-OtBu in the solvent C to obtain a solution with a concentration of 0.01-0.3 g/mL, and an addition amount of the DIPEA is 2-3 eq of moles, and an addition amount of the NCS is 1.1-1.5 eq; and in the step B, an addition amount of the Fmoc-*D*-Cys-OH is 1.1-1.5 eq.

12. A synthetic method for an etelcalcetide, comprising the following steps:
S1, synthesizing Fmoc-*D*-Cys(S-S-Boc-*L*-Cys(OtBu))-OH by the synthetic method for the etelcalcetide intermediate of any one of claims 1 to 7; and
S2, enabling NH₂-*D*-Ala-*D*-Arg-*D*-Arg-*D*-Arg-*D*-Ala-*D*-Arg to react with the Fmoc-*D*-Cys(S-S-Boc-*L*-Cys(OtBu))-OH, and removing Fmoc, to obtain the etelcalcetide after acetylation.

13. The synthetic method of claim 12, wherein in the S2, the NH₂-*D*-Ala-*D*-Arg-*D*-Arg-*D*-Arg-*D*-Ala-*D*-Arg is NH₂-*D*-Ala-*D*-Arg-*D*-Arg-*D*-Arg-*D*-Ala-*D*-Arg-resin hexapeptide.

14. The synthetic method of claim 12, wherein the S2 comprises the following steps: linking the NH₂-*D*-Ala-*D*-Arg-*D*-Arg-*D*-Arg-*D*-Ala-*D*-Arg-resin hexapeptide by using an amino resin according to a method of solid-phase synthesis, activating the Fmoc-*D*-Cys (S-S-Boc-*L*-Cys(OtBu))-OH, PyBop and DIPEA for 0-10 min at 0-5 °C, controlling a temperature at 20-30 °C and reacting for 2-6 h, after the reaction, washing for 4-6 times by DMF, removing the Fmoc by 10%-20% of piperidine, to obtain peptide resin of the etelcalcetide after the acetylation.

15. The synthetic method of claim 14, wherein a ratio of Fmoc-*D*-Cys(S-S-Boc-*L*-Cys(OtBu))-OH: PyBop: DIPEA is 3:3-6:3-6.

## Patentansprüche

1. Synthetisches Verfahren für ein Etelcalcetid-Zwischenprodukt, wobei das Etelcalcetid-Zwischenprodukt Fmoc-D-Cys(S-S-Boc-*L*-Cys(OtBu))-OH ist und das synthetische Verfahren für das Etelcalcetid-Zwischenprodukt die folgenden Schritte umfasst:: Verwendung von *N*-(Boc)-*L*-Cys-OtBu als Rohmaterial und Erzeugung eines Primärprodukts 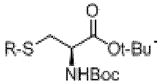 durch eine Substitutionsreaktion, wobei R S-Py oder CI ist, wodurch das Primärprodukt eine Kupplungsreaktion mit einer Fmoc-*D*-Cys-OH-Aminosäure durchführen kann, um Fmoc-*D*-Cys(S-S-Boc-*L*-Cys(OtBu))-OH zu erhalten;
wenn R S-Py ist, ist das Primärprodukt Py-S-S-Boc-*L*-Cys-OtBu, wodurch das *N*-(Boc)-*L*-Cys-OtBu die Substitutionsreaktion mit Dithiodipyridin durchführen kann, um das Primärprodukt Py-S-S-Boc-*L*-Cys-OtBu zu erhalten;
wenn R CI ist, das Primärprodukt *N*-(Boc)-*L*-Cys(S-Cl)-OtBu, wodurch *N*-(Boc)-*L*-Cys-OtBu mit NCS reagieren kann, um *N*-(Boc)-L-Cys(S-Cl)-OtBu zu synthetisieren.

2. Synthetisches Verfahren nach Anspruch 1, wobei das Primärprodukt Py-S-S-Boc-*L*-Cys-OtBu ist und das Fmoc-D-Cys(S-S-Boc-*L*-Cys(OtBu))-OH durch die folgenden Schritte hergestellt wird::
ermöglichen, dass *N*-(Boc)-*L*-Cys-OtBu die Substitutionsreaktion mit Dithiodipyridin durchführt, um ein Primärprodukt Py-S-S-Boc-*L*-Cys-OtBu zu erhalten; und
ermöglichen der Kopplung von Py-S-S-Boc-*L*-Cys-OtBu mit Fmoc-*D*-Cys-OH, um Fmoc-*D*-Cys(S-S-Boc-*L*-Cys(OtBu)-OH zu erhalten.

3. Synthetisches Verfahren nach Anspruch 2, wobei Fmoc-*D*-Cys(S-S-Boc-*L*-Cys(OtBu))-OH durch die folgenden Schritte hergestellt wird:
bei Raumtemperatur Zugabe von *N*-(Boc)-*L*-Cys-OtBu und Dithiodipyridin zu einem Lösungsmittel A, Rühren für 6-12 h und Zugabe von Wasser, Extraktion unter Verwendung eines Extraktionsmittels, Trocknen und Filtrieren einer erhaltenen organischen Phase, um nach Reinigung Py-S-S-Boc-*L*-Cys-OtBu zu erhalten;
Zugabe von Fmoc-*D*-Cys-OH und Py-S-S-Boc-*L*-Cys-OtBu zu einem Lösungsmittel B, Kontrolle der Temperatur bei 15-30 °C, Rühren und Reagieren lassen für 0,5-2 h, Waschen, Konzentrieren und Reinigen eines Reaktionssystems, um Fmoc-D-Cys(S-S-Boc-*L*-Cys(OtBu))-OH zu erhalten.

4. Synthetisches Verfahren nach Anspruch 3, wobei das Lösungsmittel A aus einem oder mehreren von DMF, NMP oder DMAc ausgewählt ist.

5. Synthetisches Verfahren nach Anspruch 3, wobei das Extraktionsmittel aus einem oder mehreren von EtOAc, MTBE oder DCM ausgewählt ist.

6. Synthetisches Verfahren nach Anspruch 3, wobei das Lösungsmittel B aus einem oder mehreren von DCM, DMF, THF, NMP oder DMAc ausgewählt ist.

7. Synthetisches Verfahren nach Anspruch 3, wobei bei der Reaktion von dem *N*-(Boc)-*L*-Cys-OtBu und dem Dithiodipyridin das Molverhältnis von dem *N*-(Boc)-*L*-Cys-OtBu und dem Dithiodipyridin 1:1,2-1:6,4 beträgt; die Konzentration von dem *N*-(Boc)-*L*-Cys-OtBu im Lösungsmittel A 0,01-0,3 g/ml beträgt; eine Konzentration des Fmoc-*D*-Cys-OH im Lösungsmittel B 0,01-0,3 g/ml beträgt; und ein Molverhältnis von dem Fmoc-*D*-Cys-OH und dem Py-S-S-Boc-*L*-Cys-OtBu 1:0,8-1,4 beträgt.

8. Synthetisches Verfahren nach Anspruch 1, wobei das Primärprodukt *N*-(Boc)-L-Cys(S-Cl)-OtBu ist und das Fmoc-*D*-Cys(S-S-Boc-*L*-Cys(OtBu))-OH durch die folgenden Schritte hergestellt wird:
ermöglichen der Reaktion von *N*-(Boc)-*L*-Cys-OtBu mit NCS, um *N*-(Boc)-*L*-Cys(S-Cl)-OtBu zu synthetisieren; und
ermöglichen der Reaktion von *N*-(Boc)-L-Cys(S-Cl)-OtBu mit Fmoc-*D*-Cys-OH, um Fmoc-D-Cys(S-S-Boc-*L*-Cys(OtBu))-OH zu erhalten.

9. Synthetisches Verfahren nach Anspruch 8, wobei das Fmoc-D-Cys(S-S-Boc-*L*-Cys(OtBu))-OH durch die folgenden Schritte hergestellt wird:
A. Lösen von Boc-*L*-Cys-OtBu in einem Lösungsmittel C, Einstellen einer Temperatur bei 0-10 °C, Zugabe von DIPEA und Zugabe von NCS in Chargen, Rühren für 4-5 h, nach Beendigung der Reaktion Filtrieren und Eluieren, um ein Filtrat zu erhalten;;
B. Halten der Temperatur bei 0-10 °C, das Boc-L-Cys-OtBu zum Filtrat hinzufügen, DIPEA hinzuzufügen, reagieren lassen und die Temperatur bei 10-30 °C halten, 0,5-2 h rühren; und das Reaktionssystem waschen, konzentrieren und reinigen um das Fmoc-*D*-Cys(S-S-Boc-*L*-Cys(OtBu))-OH zu erhalten.

10. Synthetisches Verfahren nach Anspruch 9, wobei das Lösungsmittel C aus einem oder mehreren von DCM, THF, DMF, NMP oder DMAc ausgewählt ist..

11. Synthetisches Verfahren nach Anspruch 9, wobei in Schritt A das Boc-*L*-Cys-OtBu in dem Lösungsmittel C gelöst wird, um eine Lösung mit einer Konzentration von 0,01 bis 0,3 g/ml zu erhalten, und eine Zugabemenge des DIPEA 2 -3 Moläquivalenten und eine Zugabemenge des NCS 1,1-1,5 Moläquivalenten beträgt; und in Schritt B eine Zugabemenge des Fmoc-D-Cys-OH 1,1-1,5 Moläquivalenten beträgt.

12. Synthetisches Verfahren für ein Etelcalcetid, umfassend die folgenden Schritte:
S1, Synthetisieren von Fmoc-*D*-Cys(S-S-Boc-*L*-Cys(OtBu))-OH durch das synthetische Verfahren für das Etelcalcetid-Zwischenprodukt nach einem der Ansprüche 1 bis 7; und
S2, ermöglichen NH₂-*D*-Ala-*D*-Arg-*D*-Arg-*D*-Arg-*D*-Ala-*D*-Arg mit dem Fmoc-*D*-Cys(S-S-Boc-*L*-Cys(OtBu))-OH zu reagieren, und Entfernen von Fmoc, um nach der Acetylierung Etelcalcetid zu erhalten.

13. Synthetisches Verfahren nach Anspruch 12, wobei in S2, das NH₂-*D*-Ala-*D*-Arg-*D*-Arg-*D*-Arg-*D*-Ala-*D*-Arg NH₂-*D*-Ala-*D*-Arg-*D*-Arg-*D*-Arg-*D*-Ala-*D*-Arg-Harz-Hexapeptid ist.

14. Synthetisches Verfahren nach Anspruch 12, wobei S2 die folgenden Schritte umfasst: Verknüpfen des NH₂-*D*-Ala-*D*-Arg-*D*-Arg-*D*-Arg-*D*-Ala-*D*-Arg-Harz-Hexapeptids unter Verwendung eines Aminoharzes gemäß einem Verfahren der Festphasensynthese, Aktivieren des Fmoc-*D*-Cys (S-S-Boc-*L*-Cys(OtBu))-OH, PyBop und DIPEA für 0-10 min bei 0-5 °C, Kontrolle der Temperatur bei 20-30 °C und Reaktion für 2-6 h, nach der Reaktion 4-6-maliges Waschen mit DMF, Entfernen des Fmoc durch 10%-20% Piperidin, um nach der Acetylierung ein Peptidharz des Etelcalcetids zu erhalten.

15. Synthetisches Verfahren nach Anspruch 14, wobei das Verhältnis von Fmoc-*D*-Cys(S-S-Boc-*L*-Cys(OtBu))-OH: PyBop: DIPEA 3:3-6:3-6 beträgt.

## Revendications

1. Procédé de synthèse d'un intermédiaire d'ételcalcétide, dans lequel l'intermédiaire d'ételcalcétide est Fmoc-*D*-Cys(S-S-Boc-*L*-Cys(OtBu))-OH, et le procédé de synthèse de l'intermédiaire d'ételcalcétide comprend les étapes suivantes : utilisation de *N*-(Boc)-*L*-Cys-OtBu comme une matière première, et génération d'un produit primaire 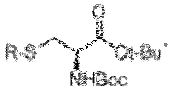 par une réaction de substitution, dans lequel R est S-Py ou Cl, permettant au produit primaire d'effectuer une réaction de couplage avec un acide aminé Fmoc-*D*-Cys-OH, afin d'obtenir le Fmoc-*D*-Cys(S-S-Boc-*L-*Cys(OtBu))-OH ;
lorsque R est S-Py, le produit primaire est Py-S-S-Boc-*L*-Cys-OtBu, ce qui permet au *N*-(Boc)-*L*-Cys-OtBu d'effectuer la réaction de substitution avec la dithiodipyridine pour obtenir un produit primaire Py-S-S-Boc-*L*-Cys-OtBu ;
lorsque R est Cl, le produit primaire est *N*-(Boc)-*L-*Cys(S-Cl)-OtBu, ce qui permet à *N*-(Boc)-*L*-Cys-OtBu de réagir avec NCS pour synthétiser *N*-(Boc)-*L*-Cys(S-Cl)-OtBu.

2. Procédé de synthèse de la revendication 1, dans lequel le produit primaire est Py-S-S-Boc-*L*-Cys-OtBu, et le Fmoc-*D*-Cys(S-S-Boc-*L*-Cys(OtBu))-OH est préparé par les étapes suivantes :
permettre au *N*-(Boc)-*L*-Cys-OtBu d'effectuer la réaction de substitution avec la dithiodipyridine pour obtenir un produit primaire Py-S-S-Boc-*L*-Cys-OtBu ; et
permettre de coupler le Py-S-S-Boc-*L*-Cys-OtBu avec le Fmoc-*D*-Cys-OH pour obtenir le Fmoc-*D*-Cys(S-S-Boc-*L-*Cys(OtBu)-OH.

3. Procédé de synthèse de la revendication 2, dans lequel le Fmoc-*D*-Cys(S-S-Boc-*L*-Cys(OtBu))-OH est préparé par les étapes suivantes :
à température ambiante, ajouter du *N*-(Boc)-*L*-Cys-OtBu et de la dithiodipyridine à un solvant A, agiter pendant 6 à 12 h, et ajouter de l'eau, extraire à l'aide d'un agent d'extraction, sécher et filtrer une phase organique obtenue, pour obtenir le Py-S-S-Boc-*L*-Cys-OtBu après purification ;
ajouter le Fmoc-*D*-Cys-OH et le Py-S-S-Boc-*L*-Cys-OtBu à un solvant B, réguler une température de 15 à 30 °C, agiter et mettre à réagir pendant 0,5 à 2 h, laver, concentrer et purifier un système de réaction, de manière à obtenir le Fmoc-*D*-Cys(S-S-Boc-*L*-Cys(OtBu))-OH.

4. Procédé de synthèse selon la revendication 3, dans lequel le solvant A est choisi parmi un ou plusieurs parmi DMF, NMP ou DMAc.

5. Procédé de synthèse selon la revendication 3, dans lequel l'agent d'extraction est choisi parmi un ou plusieurs parmi EtOAc, MTBE ou DCM.

6. Procédé de synthèse selon la revendication 3, dans lequel le solvant B est choisi parmi un ou plusieurs parmi DCM, DMF, THF, NMP ou DMAc.

7. Procédé de synthèse de la revendication 3, dans lequel, dans la réaction du *N*-(Boc)-*L*-Cys-OtBu et de la dithiodipyridine, le rapport molaire du *N*-(Boc)-*L*-Cys-OtBu et de la dithiodipyridine est de 1:1,2-1:6.4 ; la concentration du *N*-(Boc)-*L*-Cys-OtBu dans le solvant A est de 0,01-0,3 g/ml ; la concentration du Fmoc-*D*-Cys-OH dans le solvant B est de 0,01-0,3 g/ml ; et le rapport molaire du Fmoc-*D*-Cys-OH et du Py-S-S-Boc-*L*-Cys-OtBu est de 1: 0,8-1,4.

8. Procédé de synthèse de la revendication 1, dans lequel le produit primaire est *N*-(Boc)-*L*-Cys(S-Cl)-OtBu, et le Fmoc-*D*-Cys(S-S-Boc-*L*-Cys(OtBu))-OH est préparé par les étapes suivantes :
permettre à *N*-(Boc)-*L*-Cys-OtBu de réagir avec NCS pour synthétiser *N*-(Boc)-*L*-Cys(S-Cl)-OtBu ; et
permettre au *N*-(Boc)-*L*-Cys(S-Cl)-OtBu de réagir avec le Fmoc-*D*-Cys-OH pour obtenir le Fmoc-*D*-Cys(S-S-Boc-*L-*Cys(OtBu))-OH.

9. Procédé de synthèse de la revendication 8, dans lequel le Fmoc-*D*-Cys(S-S-Boc-*L*-Cys(OtBu))-OH est préparé par les étapes suivantes :
A. dissoudre Boc-*L*-Cys-OtBu dans un solvant C, réguler une température à 0 à 10 °C, ajouter DIPEA, et ajouter NCS par lots, agiter pendant 4 à 5 h, après avoir terminé la réaction, filtrer et éluer, pour obtenir un filtrat ; et
B. réguler une température de 0 à 10 °C, ajouter le Boc-*L*-Cys-OtBu au filtrat, ajouter DIPEA, mettre à réagir et réguler la température à 10 à 30 °C, agiter pendant 0,5 à 2 h ; et laver, concentrer et purifier un système réactionnel pour obtenir le Fmoc-*D*-Cys(S-S-Boc-L-Cys(OtBu))-OH.

10. Procédé de synthèse selon la revendication 9, dans lequel le solvant C est choisi parmi un ou plusieurs parmi DCM, THF, DMF, NMP ou DMAc.

11. Procédé de synthèse selon la revendication 9, dans lequel, à l'étape A, on dissout le Boc-*L*-Cys-OtBu dans le solvant C pour obtenir une solution d'une concentration de 0,01-0,3 g/ml, et une quantité d'ajout du DIPEA est de 2-3 éq de moles, et une quantité d'ajout de NCS est de 1,1-1,5 éq ; et à l'étape B, une quantité d'ajout de Fmoc-*D*-Cys-OH est 1,1-1,5 éq.

12. Procédé de synthèse d'un ételcalcétide, comprenant les étapes suivantes :
S1, synthétiser Fmoc-*D*-Cys(S-S-Boc-*L*-Cys(OtBu))-OH par le procédé de synthèse de l'intermédiaire d'ételcalcétide de l'une quelconque des revendications 1 à 7 ; et
S2, permettre à NH₂-*D*-Ala-*D*-Arg-*D*-Arg-*D*-Arg-*D*-Ala-*D*-Arg de réagir avec le Fmoc-*D*-Cys(S-S-Boc-*L*-Cys(OtBu))-OH, et éliminer le Fmoc, pour obtenir l'ételcalcétide après acétylation.

13. Procédé de synthèse selon la revendication 12, dans le S2, le NH₂-*D*-Ala-*D*-Arg-*D*-Arg-*D*-Arg-*D*-Ala-*D*-Arg est l' hexapeptide NH₂-*D*-Ala-*D*-Arg-*D*-Arg-*D*-Arg-*D*-Ala-*D*-Arg-résine.

14. Procédé de synthèse de la revendication 12, dans lequel le S2 comprend les étapes suivantes : lier l'hexapeptide NH₂-*D*-Ala-*D*-Arg-*D*-Arg-*D*-Arg-*D*-Ala-*D*-Arg-résine en utilisant une résine à amino selon un procédé de synthèse en phase solide, activer le Fmoc-*D*-Cys (S-S-Boc-*L*-Cys(OtBu))-OH, PyBop et DIPEA pendant 0 à 10 min à 0 à 5 °C, réguler la température à 20 à 30 °C et mettre en réaction pendant 2 à 6 h, après la réaction, laver 4 à 6 fois par du DMF, éliminer du Fmoc par 10 % à 20 % de pipéridine, pour obtenir la résine peptidique de l'ételcalcétide après l'acétylation.

15. Procédé de synthèse selon la revendication 14, dans lequel un rapport Fmoc-*D*-Cys(S-S-Boc-*L*-Cys(OtBu))-OH:PyBop:DIPEA est de 3:3-6:3-6.
